# EUROPÄISCHE PATENTANMELDUNG

(11) **EP 0 700 659 A2**
(43) Veröffentlichungstag der Anmeldung: **13.03.1996**
(21) Anmeldenummer: 95113950.0
(22) Anmeldetag: 06.09.1995
(51) Int. Cl.: A61B 5/097

(54) **Verfahren und Vorrichtung zum Ermitteln von Parametern der Atemphysiologie**

(30) Priorität: 08.09.1994 DE 9414557 U
(71) Anmelder: GOTTLIEB WEINMANN GERÄTE FÜR MEDIZIN UND ARBEITSSCHUTZ GMBH & CO., D-22525 Hamburg (DE)
(72) Erfinder: Ebers, Manfred, D-22457 Hamburg (DE); Maurer, Jörg, D-22113 Oststeinbek (DE)
(74) Vertreter: Liebelt, Rolf, Dipl.-Ing.

(57) **Zusammenfassung**

Beim Ermitteln von Parametern der Atemphysiologie mit einem Schlauchsystem, das mit den Atemöffnungen einer Person verbindbar ist und in dem Meßstellen, die dem Atemluftstrom ausgesetzt sind, wird in das Schlauchsystem Spülluft mit in bezug auf die ausgeatmete Luft niedriger relativer Feuchtigkeit im Bereich der Meßstellen eingeblasen, um die ausgeatmete Luft zu entfeuchten.

## Beschreibung

Die Erfindung betrifft ein Verfahren und eine Vorrichtung zur Ermittlung von Parametern der Atemphysiologie mit einem Schlauchsystem, das mit den Atemöffnungen einer Person verbunden ist und in dem Meßstellen, z. B. Sensoren oder Impulsleitungen, vorgesehen sind.

Parameter der Atemphysiologie geben Aufschluß über den Gesundheitszustand des Lungen-/Atemtraktes einer Person. Aus der in den Atemorganen angewärmten und angefeuchteten Luft, die in das Schlauchsystem der Meßeinrichtung ausgeatmet wird und darin abkühlt, wird Feuchtigkeit abgegeben, die sich als Kondensat an den Innenflächen des Schlauchsystems niederschlägt. Dieses Kondensat kann die Meßergebnisse verfälschen.

Aufgabe der Erfindung ist es, zur Gewährleistung zuverlässiger und reproduzierbarer Meßergebnisse bei der Ermittlung von Parametern der Atemphysiologie, die Kondensatbildung im Schlauchsystem sicher zu verhindern.

Diese Aufgabe wird bei einem Verfahren der eingangs beschriebenen Gattung dadurch gelöst, daß in das Schlauchsystem Spülluft mit in bezug auf die ausgeatmete Luft niedriger relativer Feuchtigkeit im Bereich der Meßstellen eingeblasen wird.

Mit der erfindungsgemäß in das Schlauchsystem eingebrachten Spülluft, die eine niedrige relative Luftfeuchtigkeit hat, wird die ausgeatmete Luft auf einfache Weise entfeuchtet, indem die von der Atemluft abgegebene Feuchtigkeit von der relativ trockenen Spülluft aufgenommen wird.

Bei einer zweckmäßigen Weiterbildung der Erfindung hat es sich bewährt, die dem Schlauchsystem zugeführte Menge Spülluft in Abhängigkeit von dem Volumen und der relativen Feuchtigkeit der ausgeatmeten Luft zu steuern, so daß stets die Menge Spülluft in das System eingebracht wird, die eine Kondensatbildung sicher unterbindet.

Eine Vorrichtung zur Durchführung des Verfahrens nach der Erfindung umfaßt ein mit den Atemöffnungen einer Person verbindbares Schlauchsystem mit Meßstellen, die an Anzeige-, Kontroll- und/oder Regeleinrichtungen angeschlossen sind, und ist durch mindestens einen Anschlußstutzen für eine Spülluft am Schlauchsystem im Bereich der Meßstellen gekennzeichnet.

Diese neue Vorrichtung ermöglicht die Durchführung des erfindungsgemäßen Verfahrens zum Ermitteln von Parametern der Atemphysiologie durch die Ausbildung eines Anschlußstutzens für eine Spülluftleitung am Schlauchsystem mit einfachen Mitteln. Dabei kann zur Verringerung der Baumaße und damit zur patientenfreundlichen Ausgestaltung der Vorrichtung die als Widerstandsreferenz wirkende Atemgasleitung des Schlauchsystems als Wendel ausgebildet sein, die in die Wandung eines hülsenförmigen Gehäuses im Bereich der Meßstellen eingefügt ist.

Ein Ausführungsbeispiel der Erfindung wird noch an Hand der Zeichnungen beschrieben. Es stellen dar:
- Fig. 1: eine schematische Ansicht in Richtung des Pfeiles X gemäß Fig. 3 auf einen Abschnitt eines bei der Ermittlung von Parametern der Atemphysiologie verwendeten Schlauchsystems,
- Fig. 2: eine schematische Schnittansicht längs der Linie A - A der Fig. 3,
- Fig. 3: eine schematische Schnittansicht längs der Linie B - B der Fig. 2.
- Fig. 4: eine schematische Schnittansicht entsprechend Fig. 3 durch eine andere Ausgestaltung des erfindungsgemäßen Schlauchsystems.

Der in den Zeichnungen gezeigte Abschnitt eines erfindungsgemäßen Schlauchsystems umfaßt eine Hülse 1, deren freies Ende an eine nicht gezeigte Atemmaske anschließbar ist. Am dem freien Ende der Hülse 1 gegenüberliegenden Ende der Hülse 1 sind Anschlußstutzen 2, 3 und 4 für Schläuche ausgebildet.

Über den Anschlußstutzen 3 wird die Spülluft zum Entfeuchten der ausgeatmeten Atemluft in die Hülse 1 eingeblasen. Die ein- und ausgeatmete Atemluft gelangt über den Anschlußstutzen 4 in die Hülse 1 bzw. entweicht zusammen mit der Spülluft über diesen Stutzen 4 in die Atmosphäre. An den Stutzen 4 ist eine als Widerstandsreferenz wirkende Atemgasleitung 5 angeschlossen. Der Stutzen 2 dient zur Einführung von Meßleitungen in die Hülse 1.

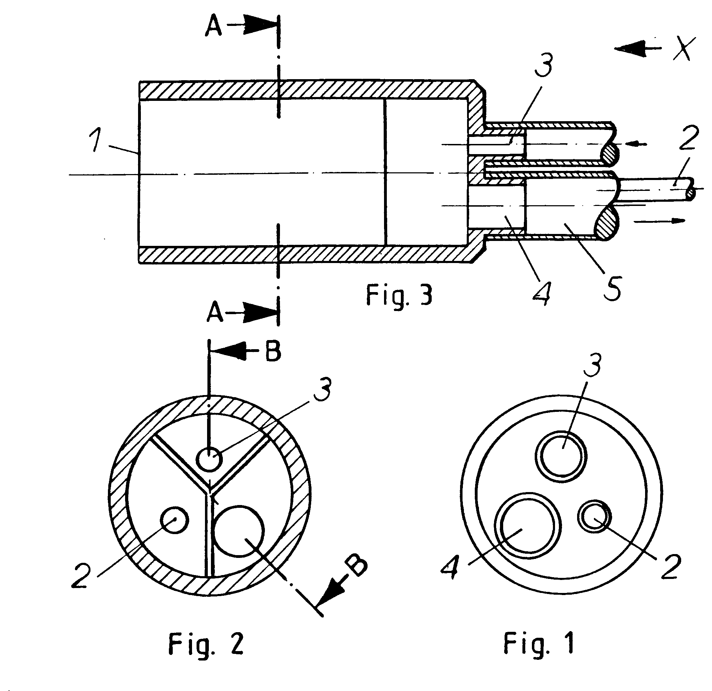

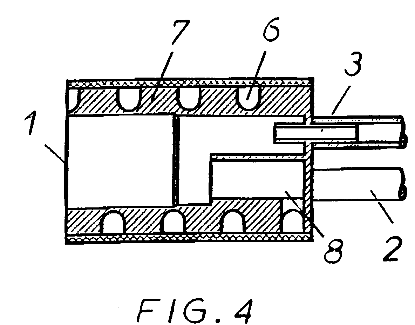

Die in Fig. 4 dargestellte Ausführung der Vorrichtung nach der Erfindung unterscheidet sich von derjenigen nach den Fig. 1 bis 3 im wesentlichen dadurch, daß der Atemschlauch als um die Hülse 1 gewickelte bzw. in die Wandung 7 der Hülse 1 eingearbeitete Wendel 6 ausgebildet ist und der Anschlußstutzen für den Atemschlauch an der Hülse 1 entfällt. Die Wendel 6 steht am freien Ende der Hülse 1 mit der Atmosphäre in Verbindung und mündet bei 8 in den Innenraum der Hülse 1.

## Patentansprüche

1. Verfahren zum Ermitteln von Parametern der Atemphysiologie mit einem Schlauchsystem, das mit den Atemöffnungen einer Person verbindbar ist und in dem Meßstellen, die dem Atemluftstrom ausgesetzt sind, vorgesehen sind, dadurch gekennzeichnet, daß in das Schlauchsystem Spülluft mit in bezug auf die ausgeatmete Luft niedriger relativer Feuchtigkeit im Bereich der Meßstellen eingeblasen wird.

2. Verfahren nach Anspruch 1, dadurch gekennzeichnet, daß die dem Schlauchsystem zugeführte Menge Spülluft in Abhängigkeit von dem Volumen und der relativen Feuchtigkeit der ausgeatmeten Luft gesteuert wird.

3. Vorrichtung zur Durchführung des Verfahrens nach Anspruch 1 oder 2, die ein mit den Atemöffnungen einer Person verbindbares Schlauchsystem mit Meßstellen umfaßt, die an Anzeige- und/oder Kontroll- und/ oder Regeleinrichtungen angeschlossen sind, dadurch gekennzeichnet, daß das Schlauchsystem im Bereich der Meßstellen mindestens einen Anschlußstutzen (3) für eine Spülluftleitung aufweist.

4. Vorrichtung nach Anspruch 3, dadurch gekennzeichnet, daß die als Widerstandsreferenz wirkende Atemgasleitung (5) des Schlauchsystems als Wendel (6) ausgebildet ist.

5. Vorrichtung nach Anspruch 4, dadurch gekennzeichnet, daß die Wendel (6) in die Wandung (7) des im Bereich der Meßstellen hülsenförmig gestalteten Abschnitts (1) des Schlauchsystems eingearbeitet ist.

6. Vorrichtung nach Anspruch 4, dadurch gekennzeichnet, daß die Wendel (6) um die Wandung (7) des im Bereich der Meßstellen hülsenförmig gestalteten Abschnitts (1) des Schlauchsystems gewickelt ist.
